# EUROPÄISCHE PATENTSCHRIFT

(11) **EP 2 124 801 B1**
(45) Veröffentlichungstag und Bekanntmachung des Hinweises auf die Patenterteilung: **07.05.2014**
(21) Anmeldenummer: 08709222.7
(22) Anmeldetag: 26.02.2008
(51) Int. Cl.: A61B 19/00

(54) **CHIRURGISCHES MARKERELEMENT, CHIRURGISCHE REFERENZIERUNGSEINHEIT UND CHIRURGISCHES NAVIGATIONSSYSTEM**
SURGICAL MARKER ELEMENT, SURGICAL REFERENCING UNIT, AND SURGICAL NAVIGATION SYSTEM
ÉLÉMENT MARQUEUR CHIRURGICAL, UNITÉ DE RÉFÉRENCEMENT CHIRURGICALE ET SYSTÈME DE NAVIGATION CHIRURGICAL

(30) Priorität: 01.03.2007 DE 102007011595
(43) Veröffentlichungstag der Anmeldung: 02.12.2009
(73) Patentinhaber: Aesculap AG, 78532 Tuttlingen (DE)
(72) Erfinder: PFEIFER, Tobias, 78662 Bösingen (DE)
(74) Vertreter: Pioch, Holger
(86) Internationale Anmeldenummer: PCT/EP2008/052328
(87) Internationale Veröffentlichungsnummer: WO 2008/104548

(56) Entgegenhaltungen:
- EP-A- 1 518 508
- WO-A-01/67979
- US-A1- 2005 075 632
- US-A1- 2005 119 639

## Beschreibung

Die vorliegende Erfindung betrifft ein chirurgisches Markerelement für eine Referenzierungseinheit eines Navigationssystems, mit einem Grundkörper, welcher eine elektromagnetische Strahlung oder Ultraschall reflektierende äußere Oberfläche aufweist, und einer Verbindungseinrichtung zum Verbinden des Markerelements mit der Referenzierungseinheit, wobei die Verbindungseinrichtung (64) derart angeordnet und ausgebildet ist, dass sie in Folge sich ändernder Umgebungsbedingungen bei einer Sterilisation oder Aufbereitung ihre Form irreversibel ändert oder zerstört wird.

Ferner betrifft die vorliegende Erfindung eine chirurgische Referenzierungseinheit, deren Position mit einem chirurgischen Navigationssystem detektierbar ist, mit mindestens einem chirurgischen Markerelement.

Des Weiteren betrifft die vorliegende Erfindung ein chirurgisches Navigationssystem mit mindestens einer mindestens drei Markerelemente umfassenden Referenzierungseinheit und mit mindestens einer Nachweisvorrichtung zum Detektieren der Position der Referenzierungseinheit, wobei die Referenzierungseinheit mindestens ein chirurgisches Markerelement umfasst.

Markerelemente der eingangs beschriebenen Art werden als sogenannte passive Markerelemente zur Ausbildung chirurgischer Referenzierungseinheiten verwendet, deren Position und Lage im Raum mittels eines chirurgischen Navigationssystems der eingangs beschriebenen Art detektierbar ist. Die Markerelemente sind vorzugsweise in Form von Kugeln ausgebildet, die eine äußere Oberfläche aufweisen, welche elektromagnetische Strahlung und/oder Ultraschall reflektieren kann. Der Einfachheit halber soll nachfolgend auch dann, wenn nur Strahlung oder elektromagnetische Strahlung explizit genannt ist, auch stets Ultraschall als optionaler Informationsträger verstanden werden. Die jeweilige Strahlung wird vorzugsweise vom Navigationssystem ausgesandt und die am Markerelement reflektierte Strahlung wird wiederum mit dem Navigationssystem detektiert. Beispielsweise durch Laufzeitmessungen der Strahlung kann dann die Position des jeweiligen Markerelements im Raum bestimmt werden.

Problematisch ist bei Markerelementen der eingangs beschriebenen Art, dass die Messgenauigkeit durch die Reinigung und Sterilisation der Markerelemente stark beeinflusst wird, falls es sich bei diesen um Einmalartikel handelt. Insbesondere kann bei diesen die elektromagnetische Strahlung oder Ultraschall reflektierende äußere Oberfläche beschädigt werden. Dies führt zu inhomogenen Reflexionseigenschaften. Insbesondere können sich die Eigenschaften unterschiedlicher, nur zur einmaligen Verwendung bestimmter Markerelemente an derselben Referenzierungseinheit beim Durchlaufen von Aufbereitungszyklen ändern. Dadurch entsteht insgesamt ein undefinierter Zustand, welcher zu signifikanten Genauigkeitseinbußen durch Verwendung ein- oder mehrfach sterilisierter Einweg-Markerelemente führt. Zudem sind die konkreten Genauigkeitseinbußen nur schwer beziehungsweise gar nicht abschätzbar.

Aus der EP 1 518 508 A1 ist eine chirurgische Referenzierungseinheit bekannt, die aus kostengünstigen Materialien hergestellt sein kann, welche einer Reinigung und Sterilisation nicht standhalten. Ferner ist in der WO 01/67979 A1 ein automatisches Kalibrierungssystem für computerunterstützte chirurgische Instrumente beschrieben.

Es ist daher Aufgabe der vorliegenden Erfindung, ein chirurgisches Markerelement, eine chirurgische Referenzierungseinheit und ein chirurgisches Navigationssystem der eingangs beschriebenen Art so zu verbessern, dass eine hochpräzise Positionsbestimmung für jedes Markerelement sichergestellt wird.

Diese Aufgabe wird bei einem chirurgischen Markerelement der eingangs beschriebenen Art erfindungsgemäß dadurch gelöst, dass der Grundkörper eine Aufnahme zum mindestens teilweisen Aufnehmen der Verbindungseinrichtung aufweist oder dass die Verbindungseinrichtung eine Aufnahme aufweist, in welche ein Vorsprung des Grundkörpers ganz oder teilweise eingeführt werden kann.

Ein derartiges Markerelement eignet sich insbesondere hervorragend als Einmalartikel. Durch die Formänderung beziehungsweise Zerstörung der Verbindungseinrichtung kann sichergestellt werden, dass die nur zum einmaligen Gebrauch vorgesehenen Markerelemente nicht mehrfach verwendet werden. Da bei Markerelementen, die nur zum einmaligen Gebrauch bestimmt sind, in Folge einer Sterilisation die äußere, elektromagnetische Strahlung oder Ultraschall reflektierende Oberfläche sehr häufig und undefiniert beschädigt wird, stellt das erfindungsgemäß weitergebildete chirurgische Markerelement sicher, dass es zum Beispiel nach einem Wiederaufbereitungszyklus nicht mehr verwendbar ist, denn die verformte beziehungsweise zerstörte Verbindungseinrichtung lässt sich dann nicht mehr in gewünschter Weise mit der Referenzierungseinheit verwenden. So lassen sich insbesondere die bekannten Genauigkeitseinbußen bei mehrfach verwendeten Markern für die Einmalbenutzung sicher vermeiden. Durch die erfindungsgemäße Weiterbildung ist sichergestellt, dass nur für zur Einmalverwendung vorgesehene Markerelemente auch tatsächlich nur einmal verwendet werden können. Besonders einfach wird der Aufbau des chirurgischen Markerelements dadurch, dass der Grundkörper eine Aufnahme zum Aufnehmen der Verbindungseinrichtung aufweist. Die Verbindungseinrichtung kann so mindestens teilweise in den Grundkörper eingeführt werden. Selbstverständlich wäre es auch denkbar, die Verbindungseinrichtung mit einer Aufnahme zu versehen, in welche beispielsweise ein Vorsprung des Grundkörpers ganz oder teilweise eingeführt werden kann.

Günstig ist es, wenn die Verbindungseinrichtung mindestens teilweise aus einem Material hergestellt ist, welches in Folge sich ändernder Umgebungsbedingungen seine Form oder seinen Aufbau irreversibel ändert oder zerstört wird. Durch die Wahl eines solchen Materials kann die Verbindungseinrichtung beim Durchlaufen beispielsweise eines Aufbereitungszyklusses gezielt ausgeschaltet werden, so dass eine Wiederverwendung des zum einmaligen Gebrauch bestimmten chirurgischen Markerelements ausgeschlossen werden kann.

Vorzugsweise ändert das Material seine Form oder seinen Aufbau oder wird zerstört auf Grund einer Druck-, Temperatur- und/oder Feuchtigkeitsänderung in einer Umgebung des Markerelements. Beispielsweise kann so in Folge einer Temperaturänderung, beispielsweise beim Durchlaufen eines Sterilisationszyklusses, das Material derart verformt oder sein Aufbau irreversibel geändert werden oder das Material zerstört werden, dass eine Wiederverwendung des chirurgischen Markerelements unmöglich wird.

Besonders günstig ist es, wenn das Material seine Form oder seinen Aufbau irreversibel ändert oder zerstört wird in Folge einer Überschreitung mindestens eines vorgegebenen Grenzwerts einer zur Beschreibung von Umgebungsbedingungen geeigneten physikalischen Größe. Beispielsweise kann es sich hier um einen Temperaturgrenzwert, einen Feuchtigkeitsgrenzwert oder einen Druckgrenzwert handeln, denn Umgebungsbedingungen lassen sich beispielsweise durch Angabe von Druck, Temperatur und/oder einer absoluten oder relativen Feuchtigkeit charakterisieren.

Besonders einfach und kostengünstig wird der Aufbau des chirurgischen Markerelements, wenn die Verbindungseinrichtung mindestens teilweise aus einem Kunststoff hergestellt ist. Kunststoffe können insbesondere derart ausgebildet sein, dass sie ihre Form oder ihren Aufbau, beispielsweise auch ihre chemische Struktur, irreversibel ändern oder ganz zerstört werden.

Vorteilhafterweise ist der Kunststoff ein thermoplastischer Kunststoff. Ein thermoplastischer Kunststoff eignet sich hervorragend, um seine Form zu ändern, wenn er einer Temperatur oberhalb eines vorgegebenen Grenzwerts ausgesetzt wird, beispielsweise einer Temperatur im Fließbereich des thermoplastischen Kunststoffs. Durch eine entsprechende Temperaturerhöhung kann der thermoplastische Kunststoff seine Form nicht beibehalten und fängt an zu fließen, wodurch er seine Form ändert. So lässt sich insbesondere eine Verbindungseinrichtung des chirurgischen Markerelements ganz oder teilweise unbrauchbar machen.

Günstig ist es, wenn der thermoplastische Kunststoff einen Fließtemperaturbereich in einem Bereich von 50°C bis 150°C aufweist. So wird sichergestellt, dass die Verbindungseinrichtung sich beim Durchlaufen eines Sterilisationszyklusses, bei welchem zum Beispiel beim Autoklavieren Temperaturen von etwa 134°C erreicht werden, in gewünschter Weise ändert, um eine Wiederverwendung des chirurgischen Markerelements ausschließen zu können.

Damit eine gewünschte Funktionalität des chirurgischen Markerelements individuell angepasst werden kann, ist es vorteilhaft, wenn die Verbindungseinrichtung aus einem anderen Material hergestellt ist als der Grundkörper. Damit kann gezielt zum Beispiel nur die Verbindungseinrichtung in ihrer Funktionalität durch geänderte Umgebungsbedingungen verändert werden, nicht jedoch der Grundkörper.

Um die Herstellung des chirurgischen Markerelements zu vereinfachen, ist es günstig, wenn die Verbindungseinrichtung form- und/oder kraftschlüssig mit dem Grundkörper verbunden ist. Beispielsweise können die Verbindungseinrichtung und der Grundkörper miteinander verschraubt, verklebt und/oder verschweißt sein. Es ist so zudem möglich, den Grundkörper und die Verbindungseinrichtung auf einfache Weise aus unterschiedlichen Materialien herzustellen.

Vorzugsweise sind der Grundkörper und die Verbindungseinrichtung unlösbar miteinander verbunden. So kann verhindert werden, dass der Grundkörper und die mit diesem in definierter Weise verbundene Verbindungseinrichtung sich wieder voneinander lösen können. Dadurch kann eine vom Hersteller des chirurgischen Markerelements nach Herstellung festgestellte Genauigkeit mindestens für eine Einmalnutzung des Markerelements gewährleistet werden. Günstigerweise ist die Aufnahme in Form eines Sacklochs ausgebildet. Ein solches lässt sich beispielsweise bereits bei der Herstellung des Grundkörpers, zum Beispiel durch Spritzgießen, vorsehen oder nachträglich durch Bearbeitung mit einem Werkzeug ausbilden.

Der Aufbau des chirurgischen Markerelements vereinfacht sich weiter, wenn das Sackloch hohlzylindrisch geformt ist.

Vorteilhaft ist es, wenn die Verbindungseinrichtung mindestens ein erstes Verbindungselement umfasst zum in Eingriff Bringen mit einem korrespondierenden zweiten Verbindungselement einer Referenzierungseinheit. So kann das chirurgische Markerelement definiert mit einer Referenzierungseinheit verbunden werden.

Besonders einfach wird der Aufbau des chirurgischen Markerelements, wenn das mindestens eine erste Verbindungselement in Form eines Rastelements ausgebildet ist. Das Markerelement kann so mit einer Referenzierungseinheit durch einfaches Verrasten verbunden werden. Insbesondere kann es so beispielsweise auf ein zweites Verbindungselement einer Referenzierungseinheit aufgeclipst werden.

Vorteilhafterweise ist das Rastelement in Form eines in radialer Richtung auf eine Längsachse der Aufnahme hin weisenden Vorsprungs ausgebildet. Zum Verrasten mit einem korrespondierenden Verbindungselements einer Referenzierungseinheit kann dieses dann beispielsweise in eine entsprechende Rastausnehmung des Verbindungselements eintauchen.

Günstig kann es ferner sein, wenn das mindestens eine erste Verbindungselement in Form eines Innengewindes ausgebildet ist. Dies ermöglicht es, das chirurgische Markerelement mit einem korrespondierenden Verbindungselement der Referenzierungseinheit zu verschrauben.

Vorzugsweise umfasst die Verbindungseinrichtung einen Träger, an welchem das mindestens eine erste Verbindungselement angeordnet ist. Mit dem Träger kann insbesondere eine Stabilität der Verbindungseinrichtung in gewünschter Weise angepasst werden.

Besonders einfach herzustellen ist das Markerelement, wenn der Träger in Form einer Hülse ausgebildet ist. Eine solche kann beispielsweise kraftund/oder formschlüssig in einer hohlzylindrischen Ausnehmung des Grundkörpers gehalten sein.

Um den mit der Verbindungseinrichtung verbundenen Grundkörper zu schützen, ist vorzugsweise ein Ende des Trägers verschlossen.

Grundsätzlich wäre es denkbar, die Verbindungseinrichtung aus zwei oder mehr Teilen herzustellen. Die Stabilität wird jedoch deutlich erhöht, wenn die Verbindungseinrichtung einstückig ausgebildet ist. Beispielsweise kann die Verbindungseinrichtung so aus einem Kunststoff als ein einziges Bauteil gespritzt werden.

Auch wenn es prinzipiell möglich wäre, den Grundkörper aus einem einzigen Teil herzustellen, so ist es jedoch deutlich einfacher, das Markerelement auszubilden, wenn der Grundkörper mindestens zwei miteinander verbundene Grundkörperteile umfasst. Diese können insbesondere auch aus unterschiedlichen Materialien geformt sein.

Denkbar wäre es, die mindestens zwei Grundkörperteile lösbar miteinander zu verbinden. Vorzugsweise sind diese jedoch unlösbar miteinander verbunden, was die Stabilität des Markerelements deutlich erhöht.

Vorteilhafterweise sind die mindestens zwei Grundkörperteile miteinander verklebt und/oder verschraubt. So lässt sich insgesamt eine besonders sichere und definierte Verbindung der zwei Grundkörperteile miteinander herstellen.

Eine Positionsbestimmung des Markerelements im Raum mit einem Navigationssystem ist besonders einfach und präzise, wenn der Grundkörper kugelförmig ausgebildet ist.

Vorteilhafterweise weist die äußere Oberfläche die Form einer Kegeloberfläche auf. Unabhängig von einer Form des Grundkörpers hat eine äußere Oberfläche in Form einer Kugeloberfläche den Vorteil, dass eine Positionsbestimmung des Markerelements mit einem Navigationssystem hochgenau möglich ist.

Grundsätzlich wäre es denkbar, den Grundkörper aus einem Material herzustellen, welches elektromagnetische Strahlung und/oder Ultraschall reflektiert. Wesentlich kostengünstiger ist ein Markerelement jedoch herzustellen, wenn der Grundkörper von einer Hülle umschlossen ist und wenn die Hülle die äußere Oberfläche definiert. Auf diese Weise muss nur die Hülle aus einem Material hergestellt werden, welches geeignet ist, elektromagnetische Strahlung und/oder Ultraschall zu reflektieren.

Um eine definierte Positionsbestimmung mit dem Markerelement sicherzustellen, ist es günstig, wenn die Hülle mit dem Grundkörper unlösbar verbunden ist.

Vorteilhafterweise weist die Hülle eine reflektierende Beschichtung auf, die die äußere Oberfläche definiert. So kann mit dem Markerelement elektromagnetische Strahlung und/oder Ultraschall in definierter Weise reflektiert werden, um die Position des Markerelements im Raum zu bestimmen.

Günstig ist es, wenn die Hülle mindestens zwei Beschichtungselemente umfasst. Zwei Beschichtungselemente vorzusehen erleichtert die Herstellung des Markerelements, da die zwei Beschichtungselemente den Grundkörper auf einfache Weise vollständig umschließen können.

Insbesondere dann, wenn der Grundkörper in Form einer Kugel ausgebildet ist, ist es vorteilhaft, wenn die mindestens zwei Beschichtungselemente in Form eines Teils einer Kugelschale ausgebildet sind. Derartige Beschichtungselemente können einen kugelförmigen Grundkörper formschlüssig aufnehmen.

Gemäß einer weiteren bevorzugten Ausführungsform der Erfindung kann vorgesehen sein, dass die Hülle in Form einer reflektierenden Beschichtung ausgebildet ist. Darunter ist zu verstehen, dass der Grundkörper direkt mit einer reflektierenden Beschichtung versehen werden kann, die dann den Grundkörper einhüllt und eine Hülle bildet. Zum Beispiel kann der Grundkörper mit einer reflektierenden Farbe beschichtet oder bestrichen werden.

Die eingangs gestellte Aufgabe wird ferner bei einer chirurgischen Referenzierungseinheit der eingangs beschriebenen Art erfindungsgemäß dadurch gelöst, dass der Grundkörper eine Aufnahme zum mindestens teilweisen Aufnehmen der Verbindungseinrichtung aufweist oder dass die Verbindungseinrichtung eine Aufnahme aufweist, in welche ein Vorsprung des Grundkörpers ganz oder teilweise eingeführt werden kann.

Eine derartige chirurgische Referenzierungseinheit hat den Vorteil, dass Markerelemente, die nur zur einmaligen Verwendung mit der Referenzierungseinheit vorgesehen sind, auch tatsächlich nur einmal verwendet werden können, denn ihre Verbindungseinrichtung wird erfindungsgemäß in Folge sich ändernder Umgebungsbedingungen in ihrer Form irreversibel geändert oder ganz oder zumindest derart zerstört, dass eine nochmalige Verbindung mit der Referenzierungseinheit für solche Markerelemente nicht möglich ist. Besonders einfach wird der Aufbau des chirurgischen Markerelements dadurch, dass der Grundkörper eine Aufnahme zum Aufnehmen der Verbindungseinrichtung aufweist. Die Verbindungseinrichtung kann so mindestens teilweise in den Grundkörper eingeführt werden. Selbstverständlich wäre es auch denkbar, die Verbindungseinrichtung mit einer Aufnahme zu versehen, in welche beispielsweise ein Vorsprung des Grundkörpers ganz oder teilweise eingeführt werden kann.

Günstig ist es, wenn das Markerelement eines der oben beschriebenen Markerelemente ist. Es weist dann auch die im Zusammenhang mit den oben beschriebenen Ausführungsformen erläuterten Vorteile auf.

Des Weiteren wird die eingangs beschriebene Aufgabe erfindungsgemäß bei einem chirurgischen Navigationssystem der eingangs beschriebenen Art erfindungsgemäß dadurch gelöst, dass der Grundkörper eine Aufnahme zum mindestens teilweisen Aufnehmen der Verbindungseinrichtung aufweist oder dass die Verbindungseinrichtung eine Aufnahme aufweist, in welche ein Vorsprung des Grundkörpers ganz oder teilweise eingeführt werden kann.

Ein solches Navigationssystem ermöglicht es, sicherzustellen, dass Markerelemente, die nur zur einmaligen Verwendung vorgesehen sind, zum Beispiel nach einer Aufbereitung, beispielsweise einer Reinigung in Verbindung mit nachfolgender Sterilisation, nicht mehr genutzt werden können, da ihre Verbindungseinrichtung nicht mehr geeignet ist, mit einer Referenzierungseinheit des Navigationssystems verbunden zu werden. Genauigkeitseinbußen bei der Bestimmung einer Position der Referenzierungseinheit mit dem Navigationssystem im Raum werden dadurch vermieden. Besonders einfach wird der Aufbau des chirurgischen Markerelements dadurch, dass der Grundkörper eine Aufnahme zum Aufnehmen der Verbindungseinrichtung aufweist. Die Verbindungseinrichtung kann so mindestens teilweise in den Grundkörper eingeführt werden. Selbstverständlich wäre es auch denkbar, die Verbindungseinrichtung mit einer Aufnahme zu versehen, in welche beispielsweise ein Vorsprung des Grundkörpers ganz oder teilweise eingeführt werden kann.

Günstigerweise ist das Markerelement des Navigationssystems eines der oben beschriebenen Markerelemente. Es weist dann auch die im Zusammenhang mit den oben beschriebenen Ausführungsformen von Markerelementen erläuterten Vorteile auf.

Die nachfolgende Beschreibung einer bevorzugten Ausführungsform der Erfindung dient im Zusammenhang mit der Zeichnung der näheren Erläuterung. Es zeigen:
- Figur 1:: eine schematische Darstellung eines Navigationssystems; und
- Figur 2:: eine perspektivische Darstellung einer Referenzierungseinheit mit vier Markerelementen, von denen eines in einer Schnittansicht dargestellt ist.

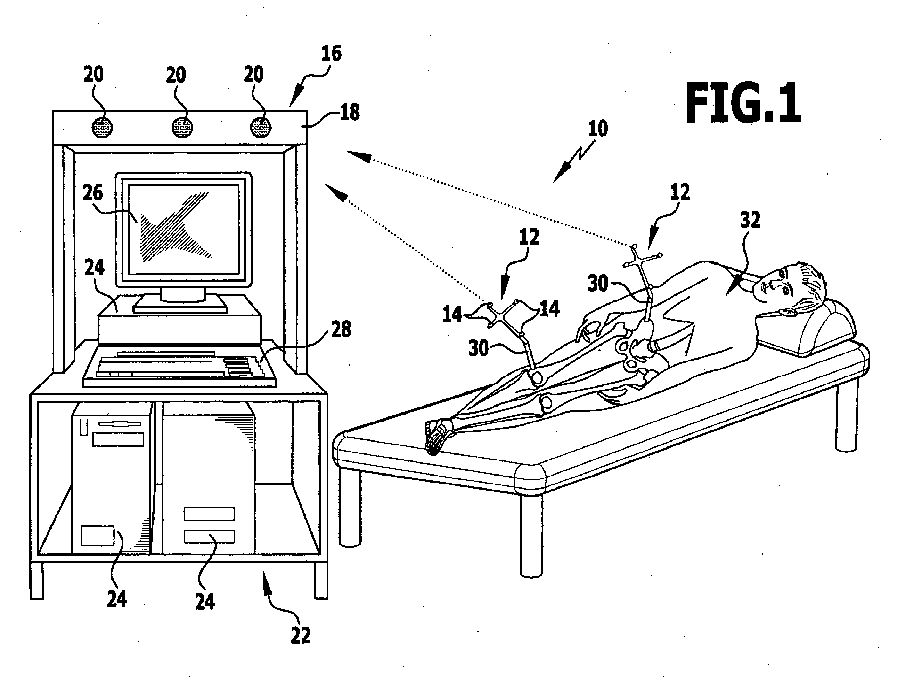

In Figur 1 ist beispielhaft ein insgesamt mit dem Bezugszeichen 10 versehenes Navigationssystem dargestellt. Es umfasst mehrere Referenzierungseinheiten 12, welche vorzugsweise mindestens drei, bei den in den Figuren dargestellten Ausführungsbeispielen jeweils vier, Markerelemente 14 umfassen.

Das Navigationssystem 10 umfasst eine Sende- und Empfangseinheit 16 zum Aussenden und Empfangen von elektromagnetischer Strahlung und/oder Ultraschall. Sie umfasst einen balkenförmigen Träger 18, an welchem drei Sender/Empfänger 20 angeordnet sind, mit welchen elektromagnetische Strahlung beziehungsweise Ultraschall ausgesandt und/oder empfangen werden können. Grundsätzlich könnten nur zwei Sender/Empfänger 20 vorgesehen sein. Um eine Genauigkeit bei der Positionsbestimmung der Referenzierungseinheiten 12 zu verbessern, können auch drei oder mehr derartige Sender/Empfänger 20 vorgesehen werden. Des Weiteren umfasst das Navigationssystem 10 eine Datenverarbeitungsanlage 22, welche bei dem in Figur 1 dargestellten Ausführungsbeispiel drei miteinander zusammengeschaltete Computer 24, einen Monitor 26 und ein Eingabegerät in Form einer Tastatur 28 umfasst. Mit der Datenverarbeitungsanlage 22 können von der Sende- und Empfangseinheit 16 erzeugte und/oder empfangene Signale verarbeitet werden, um eine Position und/oder eine Orientierung einer Referenzierungseinheit 12 im Raum zu bestimmen.

Referenzierungseinheiten 12 können insbesondere derart ausgebildet sein, dass sie mit entsprechenden Adaptern 30 zum Beispiel an einem Patienten 32 festgelegt werden können. Insbesondere können so Gelenkpositionen und Gelenkzentren des Patienten 32 ermittelt werden, indem ein Körperteil des Patienten 32, an dem ein erstes Referenzelement 12 festgelegt ist, relativ zu einem anderen Körperteil des Patienten 32 bewegt wird, an welchem eine weitere Referenzierungseinheit 12 festgelegt ist. Alternativ kann eine Referenzierungseinheit 12 auch an einem chirurgischen Instrument oder einem Werkzeug angeordnet sein, zum Beispiel unter Verwendung eines dafür geeigneten Adapters 30.

Die Referenzierungseinheit 12 umfasst einen kreuzförmigen Träger 34, welcher vier im Wesentlichen senkrecht relativ zueinander angeordnete Trägerarme 36 umfasst, die insbesondere unterschiedliche Längen aufweisen können. Jeder Trägerarm 36 trägt ein Verbindungselement in Form eines zapfenförmigen Adapters 38, welche jeweils senkrecht von einer ebenen Oberfläche 40 des Trägers 34 abstehen. Jeder Adapter 38 ist mit einer umlaufenden Ringnut 42 versehen, welche ein Rastelement bildet. Die Ringnut 42 ist konzentrisch zu einer vom Adapter 38 definierten Längsachse 44 angeordnet und teilt den Adapter 38 in zwei Teile, die in einem Längenverhältnis von etwa zwei zu drei stehen, wobei das längere Teil des Adapters 38 direkt an den Träger 34 angrenzt.

Das Markerelement 14 umfasst einen kugelförmigen Grundkörper 46, welcher zwei unlösbar miteinander verbundene Kugelhälften 48 und 50 umfasst. In der oberen Kugelhälfte 48 ist konzentrisch zu einer durch ein Zentrum des Grundkörpers 46 verlaufenden Längsachse 52 ein mit einem Innengewinde versehenes zylindrisches Sackloch 54 ausgebildet, welches sich senkrecht zu einer kreisförmigen Seitenfläche 56 in die obere Kugelhälfte 48 hinein erstreckt. Von einer ebenfalls kreisförmigen Seitenfläche 58 der unteren Kugelhälfte 50 erstreckt sich senkrecht ein mit einem Außengewinde versehener zylindrischer Zapfen 60 weg, so dass die Kugelhälften 48 und 50 miteinander verschraubt werden können. Optional können sie ferner miteinander verklebt werden, und zwar indem Klebstoff auf die ineinandergreifenden Gewinde des Sacklochs 54 und des Zapfen 60 aufgetragen wird oder alternativ beziehungsweise zusätzlich auf die Seitenflächen 56 und 58.

Die untere Kugelhälfte 50 ist mit einer Aufnahme 62 versehen, die in Form eines hohlzylindrischen Sacklochs ausgebildet ist. Es definiert eine Längsachse, die mit der Längsachse 52 des Zapfens 60 zusammenfällt. In die Aufnahme 62 ist eine Verbindungseinrichtung 64 eingesetzt, welche einen hülsenförmigen Träger 66 umfasst, welcher einen geschlossenen Boden 68 aufweist. Der Träger 66 ist in die Aufnahme 62 derart eingesetzt, dass der Boden 68 flächig an einem Boden 70 der Aufnahme 62 anliegt. Insgesamt füllt die Verbindungseinrichtung 64 die Aufnahme 62 formschlüssig aus und bildet eine Aufnahme für einen Adapter 38. Etwa auf drei fünftel der Länge des Trägers 66 parallel zur Längsachse 52 ist von einer Innenwand 72 des Trägers 66 abstehend ein radial nach innen weisender, ringförmiger Rastvorsprung 74 ausgebildet, welcher im Querschnitt eine dreieckige Form aufweist, wobei eine Spitze beziehungsweise eine ringförmige Kante des Rastvorsprungs 74 in Richtung auf die Längsachse 52 hin weist. Der Rastvorsprung 74 ist zudem derart geformt, dass er in die Ringnut 42 des Adapters 38 rastend eintauchen kann.

Die Verbindungseinrichtung 64 ist unlösbar mit dem Grundkörper 46 verbunden, vorzugsweise durch Kleben oder Schweißen. Sie ist ferner vorzugsweise aus einem Kunststoff hergestellt, insbesondere einem thermoplastischen Kunststoff, welcher einen Fließtemperaturbereich in einem Bereich von 50°C bis 150°C aufweist. Als Kunststoffe eignen sich insbesondere Polystyrol (PS), Polyethylen (PE) und Acrylnitril-Butadien-Styrol-Copolymerisat (ABS). Der Grundkörper 46, das heißt insbesondere die beiden Kugelhälften 48 und 50 können ebenfalls aus einem Kunststoff hergestellt sein. Dabei kann es sich um denselben oder auch um einen anderen Kunststoff handeln als denjenigen, aus dem die Verbindungseinrichtung 64 hergestellt ist.

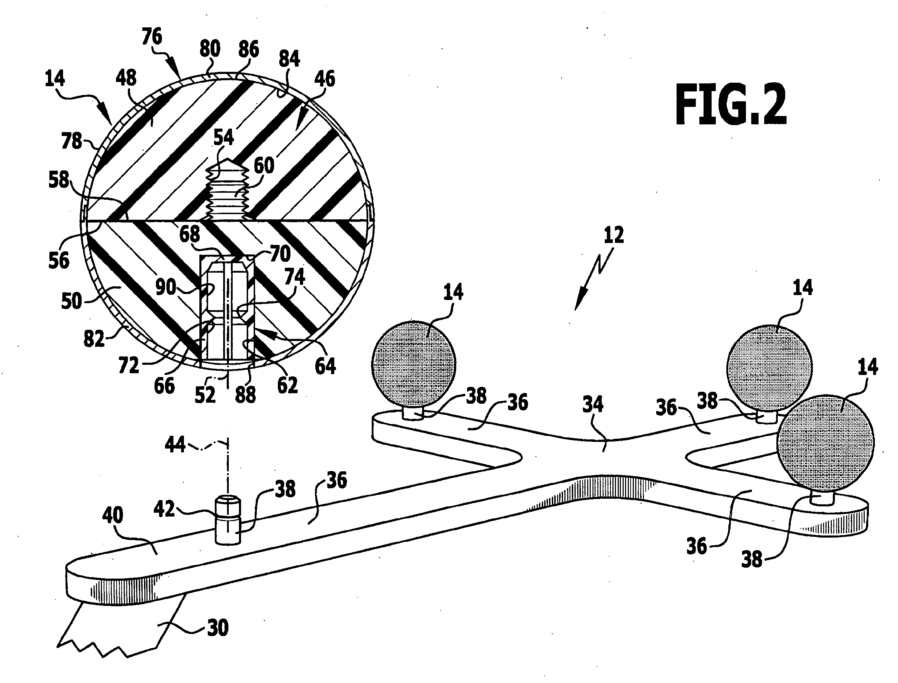

Der Grundkörper 46 ist von einer Hülle 76 umschlossen, welche eine äußere Oberfläche 78 aufweist, die derart ausgebildet ist, dass sie elektromagnetische Strahlung und/oder Ultraschall reflektieren kann. Die Hülle 76 umfasst zwei dünne Kugelschalenhälften 80 und 82, wobei eine Innenseite 84 der Hülle 76 flächig an einer, eine Kugeloberfläche definierenden Außenseite 86 des Grundkörpers 46 anliegt. Die Hülle 76 ist mit dem Grundkörper 46 vorzugsweise verklebt oder verschweißt, beispielsweise durch Ultraschall-Schweißen. Eine der beiden Kugelschalenhälften 80 oder 82, bei dem in Figur 2 dargestellten Ausführungsbeispiel die Kugelschalenhälfte 82, ist mit einer kreisförmigen Öffnung 88 versehen, die konzentrisch zur Längsachse 52 angeordnet ist. Dies ermöglicht es, den Adapter 38 durch die Öffnung 88 hindurch in die von der Verbindungseinrichtung 64 definierte Aufnahme einzuführen, und zwar so weit, bis der Rastvorsprung 74 in die Ringnut 42 einrastet.

Die Markerelemente 14 sind vorzugsweise als Einmalartikel ausgebildet, das heißt sie sind nur zur einmaligen Verwendung gedacht. Die Verbindungseinrichtung 64 jedes Markerelements 14 ist daher derart ausgebildet, dass sie in Folge sich ändernder Umgebungsbedingungen ihre Form irreversibel ändert beziehungsweise ganz zerstört wird. Dies kann beispielsweise dadurch erreicht werden, dass das Markerelement 14 einer Druck-, Temperatur- und/oder Feuchtigkeitsänderung unterworfen wird, beispielsweise im Rahmen einer Aufbereitung der Referenzierungseinheit 12, bei welcher diese gereinigt und anschließend dampfsterilisiert wird. Durch entsprechende Auswahl des Materials, aus dem die Verbindungseinrichtung 64 hergestellt ist, beispielsweise einem Kunststoff, fängt dieser bei entsprechender Erwärmung an zu fließen, so dass sich insbesondere die Innenwand 72 und/oder der Rastvorsprung 74 derart verformen können, dass der Adapter 38 nicht mehr in die von der Verbindungseinrichtung 64 definierte hohlzylindrische Ausnehmung 90 eingeführt werden kann. Folglich kann ein Markerelement 14, welches mehr als einmal verwendet werden soll, nicht mehr mit einem entsprechenden Adapter 38 der Referenzierungseinheit 12 verbunden werden. Eine Beschädigung der reflektierenden Oberfläche 78 in Folge einer Aufbereitung, welche zu inhomogenen Reflexionseigenschaften des Markerelements 14 führt, kann somit keine Genauigkeitseinbußen bewirken, die ansonsten bei Verwendung nochmals sterilisierter Markerelemente 14 auftreten können. Markerelemente, die vor einer Aufbereitung der Referenzierungseinheit 12 vom Träger 34 entfernt werden, werden somit automatisch durch entsprechende Veränderung der Verbindungseinrichtung 64 bei Durchlaufen eines Reinigungs- und/oder Sterilisationszyklusses unbrauchbar gemacht.

## Patentansprüche

1. Chirurgisches Markerelement (14) für eine Referenzierungseinheit (12) eines Navigationssystems (10), mit einem Grundkörper (46), welcher eine elektromagnetische Strahlung oder Ultraschall reflektierende äußere Oberfläche (78) aufweist, und einer Verbindungseinrichtung (64) zum Verbinden des Markerelements (14) mit der Referenzierungseinheit (12), wobei die Verbindungseinrichtung (64) derart angeordnet und ausgebildet ist, dass sie in Folge sich ändernder Umgebungsbedingungen bei einer Sterilisation oder Aufbereitung ihre Form irreversibel ändert oder zerstört wird, **dadurch gekennzeichnet, dass** der Grundkörper (46) eine Aufnahme (62) zum mindestens teilweisen Aufnehmen der Verbindungseinrichtung (64) aufweist oder dass die Verbindungseinrichtung (64) eine Aufnahme aufweist, in welche ein Vorsprung des Grundkörpers (46) ganz oder teilweise eingeführt werden kann.

2. Chirurgisches Markerelement nach Anspruch 1, **dadurch gekennzeichnet, dass** die Verbindungseinrichtung (64) mindestens teilweise aus einem Material hergestellt ist, welches in Folge sich ändernder Umgebungsbedingungen seine Form oder seinen Aufbau irreversibel ändert oder zerstört wird.

3. Chirurgisches Markerelement nach Anspruch 1 oder 2, **dadurch gekennzeichnet, dass** das Material seine Form oder seinen Aufbau irreversibel ändert oder zerstört wird in Folge einer Überschreitung mindestens eines vorgegebenen Grenzwerts einer zur Beschreibung von Umgebungsbedingungen geeigneten physikalischen Größe.

4. Chirurgisches Markerelement nach einem der voranstehenden Ansprüche, **dadurch gekennzeichnet, dass** die Verbindungseinrichtung (64) mindestens teilweise aus einem Kunststoff hergestellt ist.

5. Chirurgisches Markerelement nach einem der voranstehenden Ansprüche, **dadurch gekennzeichnet, dass** die Verbindungseinrichtung (64) aus einem anderen Material hergestellt ist als der Grundkörper (46).

6. Chirurgisches Markerelement nach einem der voranstehenden Ansprüche, **dadurch gekennzeichnet, dass** die Verbindungseinrichtung (64) form- und/oder kraftschlüssig mit dem Grundkörper (46) verbunden ist.

7. Chirurgisches Markerelement nach einem der voranstehenden Ansprüche, **dadurch gekennzeichnet, dass** die Aufnahme (62) in Form eines Sacklochs (62) ausgebildet ist.

8. Chirurgisches Markerelement nach Anspruch 7, **dadurch gekennzeichnet, dass** die Aufnahme (62) in Form eines Sacklochs (62) ausgebildet ist, wobei insbesondere das Sackloch (62) hohlzylindrisch geformt ist.

9. Chirurgisches Markerelement nach einem der voranstehenden Ansprüche, **dadurch gekennzeichnet, dass** die Verbindungseinrichtung (64) mindestens ein erstes Verbindungselement (74) umfasst zum in Eingriff Bringen mit einem korrespondierenden zweiten Verbindungselement (38) einer Referenzierungseinheit (12).

10. Chirurgisches Markerelement nach einem der voranstehenden Ansprüche, **dadurch gekennzeichnet, dass** die Verbindungseinrichtung (64) einen Träger (66) umfasst, an welchem das mindestens eine erste Verbindungselement (74) angeordnet ist.

11. Chirurgisches Markerelement nach einem der voranstehenden Ansprüche, **dadurch gekennzeichnet, dass** der Grundkörper (46) mindestens zwei miteinander verbundene Grundkörperteile (48, 50) umfasst.

12. Chirurgisches Markerelement nach einem der voranstehenden Ansprüche, **dadurch gekennzeichnet, dass** der Grundkörper (46) von einer Hülle (76) umschlossen ist und dass die Hülle (76) die äußere Oberfläche (78) definiert.

13. Chirurgisches Markerelement nach Anspruch 12, **dadurch gekennzeichnet, dass** die Hülle (76) mindestens zwei Beschichtungselemente (80, 82) umfasst, wobei insbesondere die mindestens zwei Beschichtungselemente (80, 82) in Form eines Teils (80, 82) einer Kugelschale ausgebildet sind.

14. Chirurgische Referenzierungseinheit (12), deren Position mit einem chirurgischen Navigationssystem (10) detektierbar ist, mit mindestens einem chirurgischen Markerelement (14), **dadurch gekennzeichnet, dass** das Markerelement (14) ein Markerelement (14) nach einem der voranstehenden Ansprüche ist.

15. Chirurgisches Navigationssystem (10) mit mindestens einer mindestens drei Markerelemente (14) umfassenden Referenzierungseinheit (12) und mit mindestens einer Nachweisvorrichtung (16) zum Detektieren der Position der Referenzierungseinheit (12), wobei die Referenzierungseinheit (12) mindestens ein chirurgisches Markerelement (14) umfasst, **dadurch gekennzeichnet, dass** das Markerelement (14) ein Markerelement (14) nach einem der Ansprüche 1 bis 13 ist.

## Claims

1. Surgical marker element (14) for a referencing unit (12) of a navigation system (10), comprising a base body (46) having an outer surface (78) reflecting an electromagnetic radiation or ultrasound, and comprising a connecting device (64) for connecting the marker element (14) to the referencing unit (12), the connecting device (64) being arranged and configured such that it irreversibly changes its shape or is destroyed as a result of changing environmental conditions during sterilization or treatment, **characterized in that** the base body (46) comprises a receptacle (62) for at least partially receiving the connecting device (64) or **in that** the connecting device (64) comprises a receptacle into which a projection of the base body (46) can be fully or partly introduced.

2. Surgical marker element in accordance with claim 1, **characterized in that** the connecting device (64) is made at least partially of a material which irreversibly changes its shape or its structure or is destroyed as a result of changing environmental conditions.

3. Surgical marker element in accordance with claim 1 or 2, **characterized in that** the material irreversibly changes its shape or its structure or is destroyed as a result of at least one predetermined limit value of a physical quantity suitable for describing environmental conditions being exceeded.

4. Surgical marker element in accordance with any one of the preceding claims, **characterized in that** the connecting device (64) is made at least partially of a plastic material.

5. Surgical marker element in accordance with any one of the preceding claims, **characterized in that** the connecting device (64) is made of a different material from that of the base body (46).

6. Surgical marker element in accordance with any one of the preceding claims, **characterized in that** the connecting device (64) is connected to the base body (46) with positive locking and/or force locking.

7. Surgical marker element in accordance with any one of the preceding claims, **characterized in that** the receptacle (62) is configured in the form of a blind hole (62).

8. Surgical marker element in accordance with claim 7, **characterized in that** the receptacle (62) is configured in the form of a blind hole (62), the blind hole (62) being, in particular, of hollow-cylindrical shape.

9. Surgical marker element in accordance with any one of the preceding claims, **characterized in that** the connecting device (64) comprises at least one first connecting element (74) for engagement with a corresponding second connecting element (38) of a referencing unit (12).

10. Surgical marker element in accordance with any one of the preceding claims, **characterized in that** the connecting device (64) comprises a support (66) on which the at least one first connecting element (74) is arranged.

11. Surgical marker element in accordance with any one of the preceding claims, **characterized in that** the base body (46) comprises at least two base body parts (48, 50) connected to each other.

12. Surgical marker element in accordance with any one of the preceding claims, **characterized in that** the base body (46) is enclosed by a covering (76), and **in that** the covering (76) defines the outer surface (78).

13. Surgical marker element in accordance with claim 12, **characterized in that** the covering (76) comprises at least two coating elements (80, 82), the at least two coating elements (80, 82) being, in particular, configured in the form of a part (80, 82) of a spherical shell.

14. Surgical referencing unit (12) whose position is detectable by a surgical navigation system (10), comprising at least one surgical marker element (14), **characterized in that** the marker element (14) is a marker element (14) in accordance with any one of the preceding claims.

15. Surgical navigation system (10) comprising at least one referencing unit (12) having at least three marker elements (14), and comprising at least one detection device (16) for detecting the position of the referencing unit (12), the referencing unit (12) comprising at least one surgical marker element (14), **characterized in that** the marker element (14) is a marker element (14) in accordance with any one of claims 1 to 13.

## Revendications

1. Elément marqueur chirurgical (14) pour une unité de référencement (12) d'un système de navigation (10), comprenant un corps de base (46), qui présente une surface extérieure (78) réfléchissant un rayonnement électromagnétique ou des ultrasons, et comprenant un dispositif de liaison (64) pour assurer la liaison de l'élément marqueur (14) avec l'unité de référencement (12), le dispositif de liaison (64) étant agencé et conçu de manière à modifier sa forme de façon irréversible ou de manière à être détruit sous l'effet d'une variation des conditions environnantes lors d'une stérilisation ou d'un traitement, **caractérisé en ce que** le corps de base (46) présente un logement de réception (62) destiné à recevoir au moins partiellement le dispositif de liaison (64), ou **en ce que** le dispositif de liaison (64) présente un logement de réception dans lequel peut être insérée, en totalité ou en partie, une protubérance du corps de base (46).

2. Elément marqueur chirurgical selon la revendication 1, **caractérisé en ce que** le dispositif de liaison (64) est fabriqué au moins en partie en un matériau, qui, sous l'effet de variations des conditions environnantes, modifie de manière irréversible sa forme ou sa configuration, ou est détruit.

3. Elément marqueur chirurgical selon la revendication 1 ou la revendication 2, **caractérisé en ce que** le matériau modifie de manière irréversible sa forme ou sa configuration, ou bien est détruit sous l'effet d'un dépassement d'au moins une valeur de seuil prédéterminée d'une grandeur physique adaptée à décrire des conditions environnantes.

4. Elément marqueur chirurgical selon l'une des revendications précédentes, **caractérisé en ce que** le dispositif de liaison (64) est fabriqué, au moins en partie, en une matière plastique.

5. Elément marqueur chirurgical selon l'une des revendications précédentes, **caractérisé en ce que** le dispositif de liaison (64) est fabriqué en un autre matériau que le corps de base (46).

6. Elément marqueur chirurgical selon l'une des revendications précédentes, **caractérisé en ce que** le dispositif de liaison (64) est relié par complémentarité de formes et/ou par adhérence au corps de base (46).

7. Elément marqueur chirurgical selon l'une des revendications précédentes, **caractérisé en ce que** le logement de réception (62) est réalisé sous la forme d'un trou borgne (62).

8. Elément marqueur chirurgical selon la revendication 7, **caractérisé en ce que** le logement de réception (62) est réalisé sous la forme d'un trou borgne (62), le trou borgne (62) étant notamment d'une configuration cylindrique creuse.

9. Elément marqueur chirurgical selon l'une des revendications précédentes, **caractérisé en ce que** le dispositif de liaison (64) comprend au moins un premier élément de liaison (74) destiné à être amené en prise avec un deuxième élément de liaison (38) correspondant d'une unité de référencement (12).

10. Elément marqueur chirurgical selon l'une des revendications précédentes, **caractérisé en ce que** le dispositif de liaison (64) comprend un support (66) sur lequel est agencé ledit au moins un premier élément de liaison (74).

11. Elément marqueur chirurgical selon l'une des revendications précédentes, **caractérisé en ce que** le corps de base (46) comprend au moins deux parties de corps de base (48, 50) reliées mutuellement.

12. Elément marqueur chirurgical selon l'une des revendications précédentes, **caractérisé en ce que** le corps de base (46) est enfermé dans une enveloppe (76), et **en ce que** l'enveloppe (76) définit la surface extérieure (78).

13. Elément marqueur chirurgical selon la revendication 12, **caractérisé en ce que** l'enveloppe (76) comprend au moins deux éléments de revêtement (80, 82), lesdits au moins deux éléments de revêtement (80, 82) étant notamment réalisés sous la forme d'une partie (80, 82) d'une coque sphérique.

14. Unité de référencement chirurgicale (12), dont la position peut être détectée au moyen d'un système de navigation chirurgical (10), comprenant au moins un élément marqueur chirurgical (14), **caractérisée en ce que** l'élément marqueur (14) est un élément marqueur (14) selon l'une des revendications précédentes.

15. Système de navigation chirurgical (10) comprenant au moins une unité de référencement (12) avec au moins trois éléments marqueurs (14), et comprenant au moins un dispositif de détection (16) pour détecter la position de l'unité de référencement (12), l'unité de référencement (12) comprenant au moins un élément marqueur chirurgical (14), **caractérisé en ce que** l'élément marqueur (14) est un élément marqueur (14) selon l'une des revendications 1 à 13.
